# EUROPÄISCHE PATENTSCHRIFT

(11) **EP 2 792 774 B1**
(45) Veröffentlichungstag und Bekanntmachung des Hinweises auf die Patenterteilung: **21.10.2015**
(21) Anmeldenummer: 14161572.4
(22) Anmeldetag: 25.03.2014
(51) Int. Cl.: D04B 1/26

(54) **Kompressives Rundgestrick zum Überziehen über eine ein Gelenk aufweisende Extremität**
Compressive round knitted fabric for covering an extremity with a joint
Tricot circulaire compressif destiné à être enfilé sur une extrémité présentant une articulation

(30) Priorität: 18.04.2013 DE 102013103914
(43) Veröffentlichungstag der Anmeldung: 22.10.2014
(73) Patentinhaber: medi GmbH & Co. KG, 95448 Bayreuth (DE)
(72) Erfinder:
(74) Vertreter: Lindner Blaumeier Patent- und Rechtsanwälte

(56) Entgegenhaltungen:
- FR-A1- 2 781 816
- FR-A1- 2 801 495
- US-A1- 2012 289 875

## Beschreibung

Die Erfindung betrifft ein kompressives Rundgestrick zum Überziehen über eine ein Gelenk aufweisende Extremität, aufweisend einen Strickfaden und einen elastischen Faden, die die Maschen bilden, und einen eingelegten elastischen Schussfaden.

Derartige kompressive Rundgestricke werden häufig als Bestandteil einer Kompressionstherapie als medizinisches Hilfsmittel eingesetzt. Sie dienen dazu, von außen Druck auf das Gewebe der umschlossenen Extremität zu erzeugen, um das Lymph- oder Venensystem zu entlasten. Zur Vermeidung einer Längsnaht werden derartige Kompressionsgestricke als Rundgestrick ausgeführt. Sie bestehen aus mehreren unterschiedlichen Fäden, wobei zur Maschenbildung ein Strickfaden und ein elastischer Faden dienen, wobei der elastische Faden dem Gestrick eine grundsätzliche Dehnbarkeit verleiht. Um den geforderten Druck auf die Extremität auszuüben, ist ein elastischer Schussfaden eingelegt, der durch die Maschenreihen läuft. Der Schussfaden liegt zwischen Maschenkopf und Maschenfuß.

Das kompressive Rundgestrick wird häufig am Arm oder am Bein im Bereich des Ellebogen- oder Kniegelenks getragen, also in einem Bereich, wo eine Bewegung stattfindet bzw. sich das jeweilige Gelenk streckt und winkelt. Kompressionsgestricke der in Rede stehenden Art, die hier eingesetzt werden, sind als einfache, schlauchförmige Rundgestricke ausgeführt. Infolge der Bewegung der Extremität leidet mitunter jedoch der Tragkomfort, da es im Bereich des Gelenks zur Gelenkinnenseite hin beim Abwinkeln zu einem Aufwerfen des Materials und im Bereich der Gelenkaußenseite zu einer Überdehnung des Materials kommt.

Aus US 2012/289875 A1 ist ein kompressives Rundgestrick in Form eines Ellbogenstrumpfes, der über ein Ellbogengelenk zu ziehen ist, beschrieben, aufweisend einen Strickfaden und einen elastischen Faden, die die Maschen bilden, aufweisend:
- zwei in Gestricklängsrichtung voneinander beabstandete erste Gestrickabschnitte, zwischen denen ein Gestrickbereich bestehend aus weiteren in Umfangsrichtung aneinander anschließenden unterschiedlichen Gestrickabschnitten vorgesehen ist,
- wobei der Gestrickbereich aufweist:
   -- einen zweiten Gestrickabschnitt,
   -- zwei in Umfangsrichtung an den zweiten Gestrickabschnitt anschließende dritte Gestrickabschnitte,
   -- sowie einen in Umfangsrichtung zwischen den beiden dritten Gestrickabschnitten vorgesehenen vierten Gestrickabschnitt, der glatt gestrickt ist.

Der Erfindung liegt damit das Problem zugrunde, ein kompressives Rundgestrick anzugeben, das einen verbesserten Tragekomfort bietet.

Zur Lösung des Problem ist erfindungsgemäß ein kompressives Rundgestrick zum Überziehen über eine ein Gelenk aufweisende Extremität vorgesehen, aufweisend einen Strickfaden und einen elastischen Faden, die die Maschen bilden, und einen eingelegten elastischen Schussfaden, aufweisend:
- zwei in Gestricklängsrichtung voneinander beabstandete erste Gestrickabschnitte, die mit Flottung gestrickt sind, und zwischen denen ein Gestrickbereich bestehend aus weiteren in Umfangsrichtung aneinander anschließenden unterschiedlichen Gestrickabschnitten vorgesehen ist,
- wobei der Gestrickbereich aufweist:
   -- einen zweiten Gestrickabschnitt, der mit Flottung gestrickt ist, wobei die Flottungsanzahl größer als die eines ersten Gestrickabschnitts ist,
   -- zwei in Umfangsrichtung an den zweiten Gestrickabschnitt anschließende dritte Gestrickabschnitte, die jeweils mit Flottung gestrickt sind, wobei die Flottungen einer Maschenreihe zur nächsten Maschenreihe versetzt gestrickt sind,
   -- sowie einen in Umfangsrichtung zwischen den beiden dritten Gestrickabschnitten vorgesehenen vierten Gestrickabschnitt, der glatt gestrickt ist.

Das erfindungsgemäße kompressive Rundgestrick weist diverse separate und zueinander unterschiedliche Gestrickabschnitte auf, die aufgrund ihrer besonderen Struktur dem erfindungsgemäßen Rundgestrick eine der Anatomie der Extremität angenäherte anatomische Form verleihen. Das erfindungsgemäße Rundgestrick kann auf diese Weise leicht gewinkelt ausgeführt werden, das heißt, dass sich eine leichte Knick- oder Winkelform erzeugen lässt, die das unbelastete Gestrick von Haus aus aufweist, so dass aufgrund dieser Form in Verbindung mit besonderen, aus der unterschiedlichen Strickung der einzelnen Gestrickabschnitte sich ergebenden haptischen Eigenschaften insbesondere in den Problemzonen im Bereich der Gelenkinnen- und der Gelenkaußenseite ein deutlich verbesserter Tragekomfort gegeben ist.

Hierzu sind am Rundgestrick zum einen zwei in Gestricklängsrichtung voneinander beabstandete erste Gestrickabschnitte vorgesehen. Diese werden mit einer Flottung gestrickt, das heißt, dass nicht jede Nadel eine Masche strickt, sondern je nach der gewählten Flottung in definiertem Rhythmus eine Nadel eine Masche auslässt. Diese beiden ersten Gestrickabschnitte bilden die wesentlichen, flächenmäßig größten Bereiche des Rundgestricks. Zwischen ihnen ist nun erfindungsgemäß ein Gestrickbereich vorgesehen, der in der Tragstellung im Bereich des Gelenks zu liegen kommt. Dieser Strickbereich, der das Rundgestrick umfangsmäßig vollständig umläuft und sich, gesehen in Gestricklängsrichtung, über einen Bereich von mehreren Zentimetern erstreckt, besteht aus insgesamt drei weiteren, separaten und unterschiedlich gestrickten Gestrickbereichen.

Vorgesehen ist ein zweiter Gestrickbereich, der - wie auch der erste Gestrickbereich - ebenfalls mit Flottung gestrickt ist. Er wird jedoch so gestrickt, dass die Flottungsanzahl größer ist als die Flottungsanzahl im ersten Gestrickabschnitt. Während beispielsweise im ersten Gestrickabschnitt bei einer 1 : 3-Flottung nur jede vierte Nadel eine Masche auslässt, mithin also der Gestrick-und der elastische Faden flott liegt, ist beispielsweise im zweiten Gestrickbereich bei einer 1 : 1-Flottung jede zweite Masche ausgelassen, so dass folglich eine hohe Anzahl an flott liegenden Gestrickfadenabschnitten gegeben ist. Hierüber wird ein beachtliches Volumen an flott liegendem, also nicht maschenmäßig gebundenem Fadenmaterial realisiert. Dies führt nun dazu, dass es zu einem quasi plüschartigen Aufwerfen dieses flott liegenden Fadenmaterials kommt, nachdem sich der eingelegte Schussfaden, der die kompressiven Eigenschaften verleiht, zusammenzieht, was im Bereich jeder Flottung dazu führt, dass das dort freiliegende Fadenmaterial ebenfalls zusammengezogen wird und sich dabei leicht aufwirft. Da eine hohe Flottungsanzahl gegeben ist, verbunden mit einer hohen Anzahl an Maschenreihen, kommt es folglich zu einem großflächigen Aufwerfen des flott liegenden Gestrickfadens, so dass sich eine erhabene, sich haptisch plüschartig und weich anfühlende Flächenstruktur ergibt. Infolge des leichten Zusammenziehens des Schussfadens in Umfangsrichtung kommt es insgesamt auch dazu, dass, gesehen in Gestricklängsrichtung, die anschließenden ersten Gestrickabschnitte leicht zueinander hingezogen werden, so dass sich in diesem Bereich, bezogen auf die Gestricklängsrichtung, ein leichter Knick respektive Winkel einstellt. Die Flottungen und Maschen verlaufen vorzugsweise einheitlich in Richtung der Maschenstäbchen, so dass sich Reihen aus Flottungen und benachbarten Maschen, die sich bei einer 1 : 1-Flottung abwechseln, ergeben.

Dieser zweite Gestrickabschnitt wird in der Tragstellung im Bereich der Gelenkinnenseite positioniert. Dieser Bereich ist in der Regel wenig beansprucht, die Haut ist folglich sensibel, so dass dort über die plüschartige Oberfläche eine Polsterung respektive ein haptisch angenehmer Tragekomfort verliehen wird.

Erfindungsgemäß vorgesehen sind ferner zwei in Umfangsrichtung an den zweiten Gestrickabschnitt anschließende dritte Gestrickabschnitte, die wiederum anders gestrickt sind. Sie zeichnen sich ebenfalls durch eine gestrickte Flottung aus, wobei erfindungsgemäß die Flottungen einer Maschenreihe zur nächsten Maschenreihe versetzt gestrickt sind. Das heißt, dass beispielsweise eine Flottung einer ersten Maschenreihe beim Stricken an einer ersten Nadel ausgebildet wird, an der benachbarten zweiten Reihe wird die Flottung nicht an derselben Nadel, sondern beispielsweise an der direkt benachbarten Nadel ausgebildet, so dass sich von Maschenreihe zu Maschenreihe ein Flottungsversatz beispielsweise um jeweils eine Nadel ergibt. Auf diese Weise kann eine Oberflächenstruktur ausgebildet werden, die dem Gestrick eine gewisse Stabilität verleiht, die wiederum zu einer Reduzierung der Faltenbildung führt, das heißt, dass das Gestrick in diesem Bereich quasi etwas fester ausgeführt ist, resultierend aus dem Flottungsversatz. Die versetzten Flottungen führen wiederum zu einer entsprechenden Oberflächenstruktur, nachdem auch hier der Schussfaden die jeweilige Flottung leicht zusammenzieht. Es bilden sich also quasi linienförmige Erhebungen aus, die stabilisierend sind und dem Faltenwurf entgegenwirken. Die beiden dritten Gestrickabschnitte schließen sich in Umfangsrichtung seitlich am zweiten Gestrickabschnitt an, so dass dieser in Umfangsrichtung umfasst ist.

Schließlich ist, den Gestrickbereich abschließend, ein vierter Gestrickabschnitt vorgesehen, der zwischen den beiden dritten Gestrickabschnitten liegt. Dieser vierte Gestrickabschnitt ist einfach glatt gestrickt. Hier erzeugt jede Nadel eine Masche, das heißt, dass eine sehr hohe Maschenanzahl gegeben ist. Die Maschenanzahl ist größer als die Maschenanzahl in den angrenzenden ersten Gestrickabschnitten, wo wie beschrieben mit Flottung gestrickt ist. Dies führt dazu, dass im vierten Gestrickabschnitt infolge der höheren Maschenanzahl mehr Gestrickvolumen gegeben ist, woraus wiederum ein geometrischer Effekt resultiert. Denn hierüber kann im Bereich dieser an der Gelenkaußenseite zu positionierenden Gestrickseite ein nach außen gerichteter Knick respektive eine nach außen gerichtete Dehnung erzeugt werden.

Insgesamt sind beim erfindungsgemäßen Rundgestrick also vier unterschiedlich gestrickte Gestrickbereiche vorgesehen, nämlich die beiden ersten Gestrickbereiche, die mit Flottung gestrickt sind, der zweite Gestrickbereich, der die plüschartige Oberfläche bildet, und der mit verglichen zum ersten Gestrickabschnitt niedriger Maschenanzahl, jedoch höherer Flottungsanzahl gestrickt ist, ferner die beiden dritten Gestrickabschnitte, die mit von Reihe zu Reihe versetzten Flottungen gestrickt sind und die der Stabilisierung in Umfangsrichtung dienen, sowie der vierte Gestrickabschnitt, der glatt gestrickt ist und eine sehr hohe Maschenzahl ohne Flottung aufweist. Es bildet sich resultierend aus der besonderen Ausgestaltung des den Umfang umlaufenden Gestrickbereichs folglich im Bereich der zweiten und vierten Gestrickabschnitte jeweils stricktechnisch eine Art Knick aus, im zweiten Gestrickbereich resultierend aus dem Zusammenziehen des Schussfadens infolge der hohen Flottungsanzahl, im vierten Gestrickbereich resultierend aus einer Dehnung infolge der sehr hohen Maschenzahl, also aus dem glatt gestrickten Muster.

Insgesamt wird auf diese Weise ein anatomisch geformtes, eine haptisch angenehme imitierte Plüschzone aufweisendes Gestrick realisiert, das einen sehr hohen Tragekomfort aufweist, gleichzeitig aber seine kompressiven Eigenschaften nach wie vor beibehält.

In Weiterbildung der Erfindung kann der zweite Gestrickabschnitt in ovaler respektive ellipsoider Form gestrickt sein. Diese Form hat sich als zweckmäßig im Hinblick auf die reale Anatomie im Gelenkinnenbereich, sei es dem Ellebogengelenk, sei es dem Kniegelenk, herausgestellt. Grundsätzlich wäre es aber natürlich auch denkbar, den Bereich beispielsweise rechteckig auszuführen, die ovale bzw. ellipsoide Form hat sich jedoch als bevorzugt erwiesen.

Wie bereits beschrieben schließen die beiden dritten Gestrickabschnitte beidseits an den zweiten Gestrickabschnitt in Umfangsrichtung an. Es ist dabei denkbar, dass die beiden dritten Gestrickabschnitte an den zweiten Gestrickabschnitt nur seitlich angrenzen, so dass der zweite Gestrickabschnitt, gesehen in Gestricklängsrichtung, unmittelbar an die beiden ersten Gestrickabschnitte anschließt. Zweckmäßig ist es jedoch, wenn die dritten Gestrickabschnitte den zweiten Gestrickabschnitt vollständig umgeben, das heißt, wenn der zweite Gestrickabschnitt von den dritten Gestrickabschnitten (die dann soweit umfangsmäßig gesehen zusammenhängen) eingeschlossen ist. Dies ist im Hinblick auf die über die dritten Gestrickabschnitte angestrebte Faltenreduzierung zweckmäßig.

Wie ausgeführt werden die ersten Gestrickabschnitte mit Flottung gestrickt, wobei bevorzugt eine x : y-Flottung mit x < y gestrickt ist. Das heißt, dass mindestens eine 1 : 2-Flottung, vorzugsweise eine 1 : 3-Flottung gestrickt wird, wobei auch eine 1 : 4-Flottung oder eine 2 : 5-Flottung gleichermaßen realisiert werden kann. Bevorzugt wird eine 1 : 3-Flottung gestrickt.

Auch der zweite Gestrickabschnitt ist mit einer Flottung gestrickt, wie einleitend beschrieben. Bevorzugt wird hier eine a : b-Flottung, wobei a ≤ b ist und b < y ist. Hier kann also beispielsweise mit einer 1 : 1-Flottung gestrickt werden, so dass eine hohe Flottungsanzahl gegeben ist. Auch kann beispielsweise eine 1 : 2-Flottung gestrickt werden, solange sichergestellt ist, dass b < y ist, mithin also die Flottungsanzahl in diesem Gestrickabschnitt größer ist als im ersten Gestrickabschnitt. Bevorzugt wird aber mit einer 1 : 1-Flottung gestrickt.

Wie beschrieben dienen die dritten Gestrickbereiche, sei es, dass sie komplett separiert sind, sei es, dass sie, den zweiten Gestrickbereich einschließen, zusammenhängen, der Stabilisierung des Gestricks. Hierzu ist erfindungsgemäß der Flottungsversatz von Maschenreihe zu Maschenreihe vorgesehen. Die Flottung kann dabei jeweils um eine Masche oder um zwei Maschen versetzt sein, wobei jedoch ein Versatz um jeweils eine Masche bevorzugt ist. Das heißt, dass auch hier beispielsweise mit einer 1 : 3-Flottung gestrickt wird, jedoch die Flottung um jeweils eine Masche von Reihe zu Reihe verschoben wird. Hieraus resultiert die Bildung einer leicht erhabenen Linienstruktur, resultierend aus dem Umstand, dass der elastische Schussfaden die jeweilige Flottung wieder etwas zusammenzieht. Es ist nun möglich, stetig in eine Richtung laufende erhabene "Linien" auszubilden. Bevorzugt aber wird ein Zick-Zack-Muster erzeugt, dadurch, dass die Flottung beispielsweise über drei oder vier benachbarte Maschenreihen, umfangsmäßig gesehen, in die eine Richtung versetzt wird, wonach sich die Versatzrichtung umkehrt und die Flottung über die nächsten drei oder vier Maschenreihen in die andere Richtung versetzt wird. Es bildet sich dann eine Zick-Zack-förmig verlaufende erhabene Linienstruktur aus.

Der Strickfaden selbst kann aus einer Kunstfaser, insbesondere PA, PES, PP, oder einer Naturfaser, insbesondere Baumwolle oder Seide sein, wobei bevorzugt ein PA-Faden verwendet wird. Dabei ist es denkbar, für die Bildung der ersten bis vierten Gestrickabschnitte Strickfäden mit unterschiedlicher Farbe zu verwenden. Dies ermöglicht es, ein buntes Rundgestrick zu fertigen, bei dem die unterschiedlichen Strickbereiche, die bei einheitlicher Strickfadenfarbe sich durch die Struktur unterscheiden, nunmehr auch zusätzlich durch die unterschiedlichen Farben unterscheidbar sind. Der Einsatz unterschiedlich farbiger Strickfäden lässt somit die Bildung unterschiedlichst farbiger Gestricke zu, was aus optischen Gesichtspunkten mitunter sehr ansprechend ist.

Der elastische Faden weist zweckmäßigerweise eine Elastanseele auf, die mit einem Faden aus einer Kunstfaser, insbesondere PA, PES, PP, oder einer Naturfaser, insbesondere Baumwolle oder Seide umwunden ist. Bevorzugt wird ein Umwindungsfaden verwendet, der aus dem gleichen Material ist wie der Strickfaden. Auch hier wird bevorzugt PA verwendet.

Der Schussfaden schließlich ist aus einem Elastomer, vorzugsweise auf PU-Basis. Weiterhin kann vorgesehen sein, an den Endbereichen der ersten Gestrickabschnitte elastische Bündchen anzustricken oder anzunähen. Je nach Wunsch oder Anforderung ist es also möglich, das Bündchen selbst sogleich beim Rundstricken auszubilden, alternativ kann auch ein vorgefertigtes elastisches Bündchen angenäht werden.

Das kompressive Rundgestrick gemäß der Erfindung ist bevorzugt als Armstrumpf zum Überziehen über den Bereich des Ellebogengelenks ausgeführt. Alternativ kann es auch als Beinstrumpf, auch in Form einer Strumpfhose, zum Überziehen über den Bereich des Kniegelenks ausgeführt sein. Sowohl die Haut im Innenbereich des Ellebogen- wie auch des Kniegelenks ist relativ empfindlich, da sie kaum beansprucht wird, weshalb die erfindungsgemäß dort anzuordnende Polsterung, realisiert über den zweiten Gestrickabschnitt, den Tragekomfort besonders erhöht. Da insbesondere das Ellebogengelenk einen sehr großen Knickwinkel von weit über 90°, mitunter auch weit über 120°, aufweist, aber auch das Kniegelenk mitunter beachtlich weit einknicken kann, ist für den Tragekomfort auch die Ausbildung des vierten Gestrickabschnitts und daraus resultierend insgesamt die leicht geknickte Grundform des Rundgestricks für einen hohen Tragekomfort von Vorteil.

Weitere Vorteile, Merkmale und Einzelheiten der Erfindung ergeben sich aus dem im Folgenden beschriebenen Ausführungsbeispiel sowie anhand der Zeichnungen. Dabei zeigen:
- Fig. 1: eine Prinzipdarstellung eines erfindungsgemäßen Rundgestricks einer ersten Ausführungsform,
- Fig. 2: ein Strickmuster zur Darstellung der verschiedenen Strickarten der diversen Gestrickabschnitte, und
- Fig. 3: eine Prinzipdarstellung eines erfindungsgemäßen Rundgestricks einer zweiten Ausführungsform.

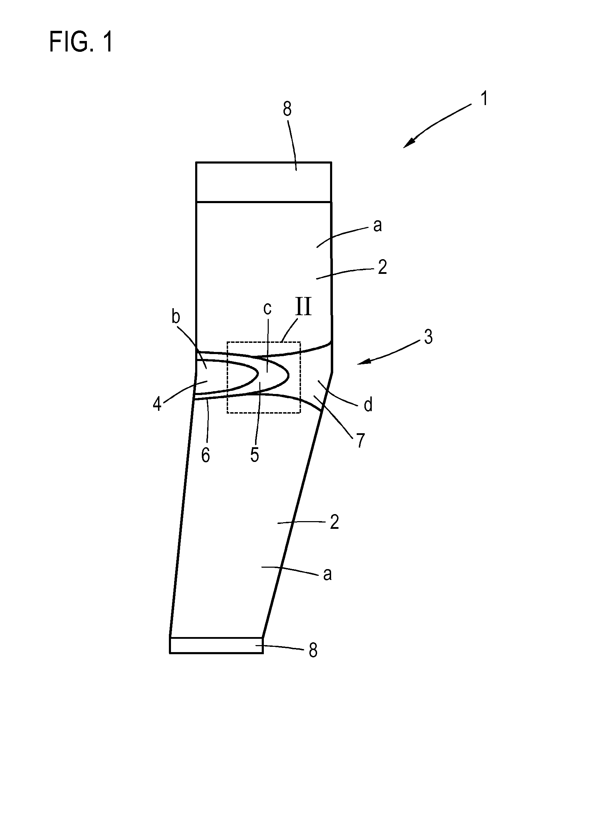

Fig. 1 zeigt ein erfindungsgemäßes kompressives Rundgestrick 1, das aus insgesamt drei Fäden gebildet ist. Zum einen zur Maschenbildung aus einem Strickfaden und einem elastischen Faden, wobei als Strickfaden bevorzugt ein PA-Faden und als elastischer Faden ein Faden mit Elastanseele und PA-Fadenumwindung verwendet wird. Ferner wird ein eingelegter elastischer Schussfaden zur Gestrickbildung verwendet. Das Rundgestrick wird auf einer Rundstrickmaschine gefertigt, so dass der Schussfaden im Gestrick zwischen den Maschenfüßen und den Maschenköpfen zweier gestrickter Reihen verläuft respektive eingelegt ist.

Das in Fig. 1 gezeigte erfindungsgemäße Rundgestrick 1, das als Armstrumpf ausgeführt ist, der über den Ober- und Unterarm, also das Ellebogengelenk überdeckend, zu tragen ist, besteht aus mehreren separaten Gestrickabschnitten. Realisiert sind zum einen zwei erste Gestrickabschnitte 2, die, bezogen auf Fig. 1, die oberen und unteren Gestrickbereiche bilden und flächenmäßig die größten Gestrickabschnitte sind. Die beiden ersten Gestrickabschnitte 2 sind mit Flottung gestrickt, bevorzugt mit einer 1 : 3-Flottung, das heißt, dass innerhalb einer Maschenreihe drei Maschen glatt gestrickt sind, während die folgende Masche ausgelassen ist und dort der Strickfaden und der elastische Faden flott liegen. Gleichwohl eingelegt respektive mitlaufend verläuft der Schussfaden.

Zwischen den beiden ersten Gestrickabschnitten 2 ist ein umfangsmäßig komplett umlaufender Gestrickbereich 3 realisiert, der aus drei weiteren separaten und unterschiedlich gestrickten Gestrickabschnitten besteht. Vorgesehen ist zum einen ein zweiter Gestrickabschnitt 4, der vorzugsweise eine ovale Form besitzt. Er ist ebenfalls mit Flottung gestrickt, er unterscheidet sich jedoch im Gestrick von den ersten Gestrickabschnitten 2 dadurch, dass die Flottungsanzahl im zweiten Gestrickabschnitt 4 deutlich größer ist als in den ersten Gestrickabschnitten 2. Bevorzugt wird mit einer 1 : 1-Flottung gestrickt, das heißt, dass sich jeweils eine glattgestrickte Masche und eine Flottung abwechseln. Da auch hier der Schussfaden eingelegt ist, kommt es dazu, dass aufgrund der Rückstellkraft, also des Zusammenziehens des Schussfadens, der Strick- und der elastische Faden innerhalb einer Flottung ebenfalls zusammengezogen respektive aufgeworfen werden. Da im zweiten Gestrickabschnitt eine sehr hohe Flottungsanzahl gegeben ist, mithin also ein hohes freiliegendes Volumen aus Gestrick- und elastischem Faden, und vorzugsweise eine 1 : 1-Flottung gegeben ist, ergibt sich folglich ein hohes, sich aufwölbendes Fadenvolumen, das diesem Gestrickabschnitt 4 eine vorstehende, erhabene und sich plüschartig anfühlende Struktur verleiht. Der Gestrickabschnitt 4 ist natürlich so gestrickt, dass sich diese erhabene Polsterung, bezogen auf die Tragstellung des Rundgestricks 1, an der Gestrickinnenseite befindet, mithin also auf der Haut aufliegt. Da die Flottungen und Maschen im zweiten Gestrickbereich von Maschenreihe zu Maschenreihe deckungsgleich liegen, ergeben sich folglich, gesehen in Gestricklängsrichtung, eine Vielzahl unmittelbar benachbarter, nur von einer Masche getrennter erhabener Linien, die, da nur über eine Masche getrennt, folglich sehr eng beieinanderliegen, so dass sich insgesamt eine quasi geschlossene, erhabene plüschartige Fläche ergibt. Die Maschenzahl im zweiten Gestrickabschnitt 4 ist folglich deutlich niedriger als in den ersten Gestrickabschnitten 2, demgegenüber ist jedoch die Flottungsanzahl deutlich höher als in den beiden ersten Gestrickabschnitten 2.

Der Gestrickbereich 3 umfasst des Weiteren zwei dritte Gestrickabschnitte 5, die - in Umfangsrichtung gesehen - beidseits an den zweiten Gestrickabschnitt 4 angrenzen. Im gezeigten Ausführungsbeispiel sind die beiden dritten Gestrickabschnitte 5 (von denen in Fig. 1 nur eine gezeigt ist, der andere befindet sich an der gegenüberliegenden Gestrickseite) miteinander über entsprechende Verbindungsabschnitte 6, die identisch gestrickt sind, verbunden, so dass sich folglich quasi ein geschlossener Gestrickabschnitt ausbildet, der den zweiten Gestrickabschnitt 4 vollständig umgibt.

Auch die dritten Gestrickabschnitte sind mit Flottung gestrickt, vorzugsweise mit einer 1 : 2-Flottung, denkbar ist aber auch eine 1 : 3-Flottung. Wichtig hierbei ist, dass die einzelnen Flottungen benachbarter Maschenreihen zueinander versetzt sind, vorzugsweise um eine Masche respektive eine Nadel, so dass sich folglich - wiederum resultierend aus dem Umstand, dass der Schussfaden die jeweilige Flottung leicht aufwirft respektive zusammenzieht - linienförmige Erhebungen ausbilden, die quasi schräg über das Gestrick verlaufen. Vorzugsweise wird dabei ein Zick-Zack-Muster gestrickt, das heißt, dass sich die Versatzrichtung der Flottungen umkehrt, also beispielsweise die Flottung über drei, vier oder fünf Maschenreihen in die eine Umfangsrichtung versetzt sind, wonach die Versatzrichtung umkehrt und die Flottung über die nächsten drei, vier oder fünf Maschenrichtungen in die andere Umfangsrichtung versetzt ist, wonach der Versatz wieder umkehrt etc. Es ergibt sich also eine Zick-Zack-Struktur, die stabilisierend, also gestrickversteifend wirkt, und einem Faltenwurf entgegenwirkt.

Schließlich ist im Gestrickbereich 3 ein vierter Gestrickabschnitt 7 vorgesehen, der an die dritten Gestrickabschnitte 5 anschließt und den Gestrickbereich 3 abschließt. In diesem Gestrickabschnitt 7 ist das Gestrick glatt gestrickt, das heißt, dass jede Nadel eine Masche strickt. Es ist hier also eine extrem hohe, also maximale Maschenanzahl gegeben, folglich auch das maximale Fadenvolumen, so dass sich, gesehen in Gestricklängsrichtung, quasi ein längsgedehnter Strickabschnitt ausbildet, der es ermöglicht, siehe Fig. 1, in diesem Bereich einen Art Knick oder Winkelstruktur auszubilden. Da wie beschrieben der zweite Gestrickabschnitt 4 mit extrem hoher Flottungsanzahl gestrickt wird und es dort aufgrund des Schussfadens zu einem starken Zusammenziehen des Gestricks, resultierend in dem plüschartigen Aufwerfen des Strickfadens kommt, ergibt sich in diesem Bereich ebenfalls ein leichter Knick. Insgesamt wird also das Gestrick stricktechnisch bedingt auf der einen Seite im zweiten Gestrickabschnitt 4 zusammengezogen, so dass sich, geometrisch gesehen, quasi eine Knickform ergibt, während an der gegenüberliegenden Seite, stricktechnisch durch die extrem hohe Maschenanzahl bedingt, sich eine Dehnung ergibt, die ebenfalls zur Ausbildung einer Knickform, die jedoch in die andere Richtung gerichtet ist, führt. Insgesamt ergibt sich folglich eine wie in Fig. 1 gezeigte, der Extremität im gestreckten Zustand leicht angenäherte geknickte Gestrickgrundform, die den Tragekomfort verbessert, verbunden mit dem Umstand, dass an der empfindlichen Gelenkinnenseite eine Polsterung, realisiert über den zweiten Gestrickabschnitt 4, positioniert werden kann.

An jedem Ende eines ersten Gestrickabschnitts 2 ist ferner ein Bündchen 8 vorgesehen, das entweder angestrickt sein kann, das aber auch, separat vorgefertigt, angenäht werden kann. Die Innenseite des Bündchens kann bei Bedarf mit einer leicht haftenden Anti-Rutsch-Beschichtung belegt sein.

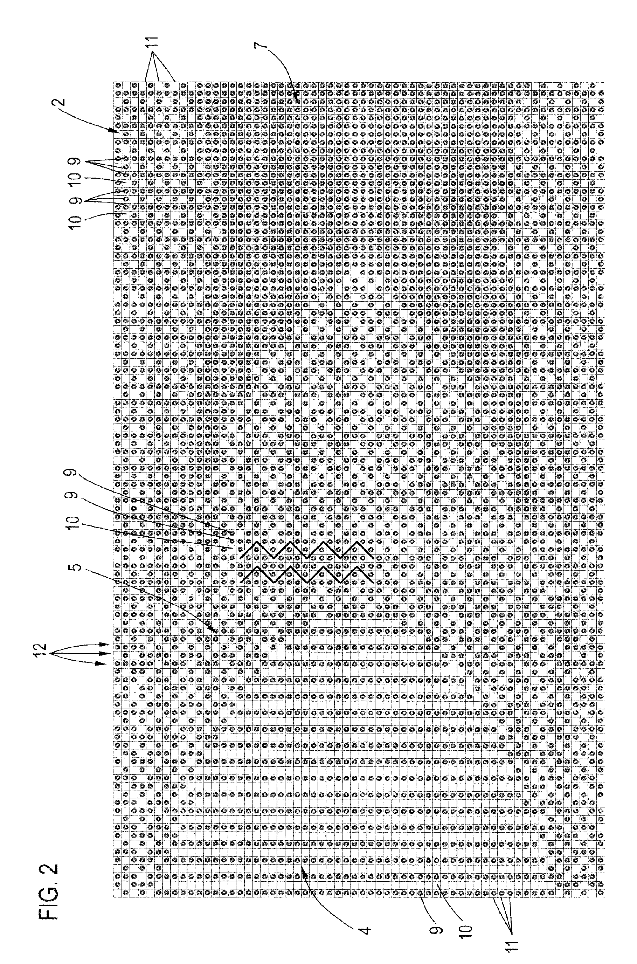

Fig. 2 zeigt ein Maschenbild als Ausschnitt (II) aus dem Rundgestrick 1 aus Fig. 1, wobei in diesem Maschenbild sämtliche Gestrickabschnitte 2, 4, 5 und 7 teilweise gezeigt sind.

Fig. 2 ist ein technisches Schnittmuster, das als x-y-Matrix mit x · y einzelnen Pixeln dargestellt ist. Jeder Pixel entspricht einer potentiellen Masche, die als solche gestrickt sein kann, oder die flott liegen kann. Jede gestrickte Masche ist mit einem "o" dargestellt, das in dem entsprechenden Pixel gezeigt ist. Nicht ausgefüllte Pixel geben an, dass dort der Faden flott liegt.

Fig. 2 zeigt zum einen die beiden ersten Gestrickabschnitte 2, die in Fig. 2 im Bereich des oberen und unteren Randes, primär nach rechts hin, dargestellt sind. Wie sich aus dem Maschenbild ergibt, ist dieser Gestrickabschnitt, also letztlich das Grundgestrick, mit einer 1 : 3-Flottung gestrickt. Ersichtlich sind jeweils drei Maschen 9 gestrickt, gefolgt von einer Flottung 10, an die wiederum drei gestrickte Maschen 9 anschließen, denen wiederum eine Flottung 10 folgt etc. Die einzelnen Maschenreihen 11 sind in diesem Bereich ersichtlich so gestrickt, dass die jeweilige Flottung 10 von Maschenreihe 11 zu Maschenreihe 11 um jeweils zwei Maschen versetzt ist.

An der gegenüberliegenden Seite in Fig. 2 ist der zweite Gestrickbereich 4 vom Maschenbild her dargestellt. Dieser zweite Strickbereich 4, der durch die spezielle Musterung als erhabener, imitierter Plüschabschnitt gestrickt ist, ist als 1 : 1-Flottung gestrickt, das heißt, dass jeweils eine Masche 9 und eine Flottung 10 einander abwechseln. Es ist also eine 1 : 1-Stäbchenbindung realisiert. Wie Fig. 2 zeigt, sind die übereinanderliegenden Maschenreihen 11 identisch gestrickt, das heißt, dass, gesehen in Gestricklängsrichtung, identische Maschenreihen 11 übereinanderliegen.

Wie bereits beschrieben führt diese Art der Stricktechnik dazu, dass der elastische Schussfaden, der wie beschrieben zwischen Maschenkopf und Maschenfuß eingelegt ist, sich im Bereich der jeweiligen Flottung 10 zusammenzieht, was dazu führt, dass das freiliegende Fadenmaterial ebenfalls zusammengezogen wird und sich leicht aufwirft. Da sämtliche Flottungen 10, siehe Fig. 2, in einer Linie liegen, entsprechendes gilt natürlich auch für die Maschen 9, führt dies dazu, dass sich quasi linien- bzw. reihenförmige Fadenerhebungen bilden. Da diese einzelnen erhabenen Reihen nur über eine Masche 9 respektive eine Maschenlinie voneinander getrennt sind, ergibt sich folglich eine nahezu geschlossene, erhabene plüschartige Oberfläche.

An den zweiten Gestrickabschnitt 4 schließen sich, wie beschrieben, beidseits die beiden dritten Gestrickabschnitte 5 an, die, wenn die Verbindungsabschnitte 6 gestrickt sind, miteinander verbunden sein können und den ovalen oder ellipsoiden zweiten Gestrickabschnitt 4 einschließen können, siehe Fig. 1. Wie dem Strickmuster aus Fig. 2 zu entnehmen ist, ist die Bindung im Gestrickabschnitt 5 als 1 : 2-Flottung realisiert, das heißt, dass hier zwei Maschen und eine Flottung, welches Bild sich fortsetzt, gestrickt ist. Dabei sind die einzelnen Maschenreihen 11 so gestrickt, dass die jeweilige Flottung 10 von Maschenreihe 11 zu Maschenreihe 11 um jeweils eine Masche respektive Nadel versetzt ist, wobei sich die Versetzungsrichtung nach jeder dritten Maschenreihe 11 umkehrt. Die Flottungen 10 verlaufen also in einem Zick-Zack-Muster, wie es in Fig. 2 exemplarisch eingezeichnet ist, siehe die beiden durchgezogenen Linien. Wie Fig. 2 zu entnehmen ist, sind eine Vielzahl solcher parallel laufender Zick-Zack-Linien realisiert.

Auch hier zieht sich im Bereich jeder einzelnen Flottung 10 der elastische Schussfaden leicht zusammen, so dass das flott liegende Fadenmaterial leicht aufgeworfen wird. Da hier jedoch in Richtung der Maschenstäbchen 12 (also vertikal zu den Maschenreihen 11) die einzelnen Flottungen 10 versetzt verlaufen und in Zick-Zack-Form gestrickt sind, kommt es dazu, dass am fertigen Gestrick die einzelnen flottungsspezifischen Fadenaufwerfungen insgesamt eine entsprechende, etwas erhabene Zick-Zack-Reihe bilden, dem Flottungsmuster folgend. Die Zick-Zack-Reihen sind, da hier eine 1 : 2-Flottung gestrickt ist, um zwei Maschen in Maschenstäbchenrichtung voneinander beabstandet, wie das Maschenbild gemäß Fig. 2 zeigt. Diese erhabenen Zick-Zack-Reihen dienen der Stabilisierung der Gestrickabschnitte 5, was einer Faltenbildung respektive einem Faltenwurf entgegenwirkt. Wenngleich Fig. 2 eine 1 : 2-Flottung zeigt, wäre es denkbar, hier natürlich auch eine 1 : 3- oder eine 2 : 4- oder 2 : 5-Flottung zu stricken. Wichtig ist lediglich, dass die jeweilige Flottung von Reihe auf Reihe um jeweils eine Nadel versetzt ist, so dass sich die Zick-Zack-Linienstruktur ausbildet.

Schießlich zeigt Fig. 2 auch das Maschenbild des vierten Gestrickabschnitts 7. Dieser Gestrickabschnitt 7 ist glatt gestrickt, das heißt, dass jede Nadel eine Masche strickt, wie dem Maschenbild eindeutig zu entnehmen ist. Hier ist also die höchste Maschenanzahl pro Flächenbereich gegeben, so dass dort ein hohes Maschen- und Fadenvolumen gegeben ist, was dazu führt, dass es in diesem Bereich zu einer Art Dehnung kommt, die die leichte Knickform, siehe Fig. 1, zur Folge hat. Demgegenüber kommt es, nachdem im zweiten Gestrickabschnitt 4 die geringste Maschenanzahl gegeben ist, jedoch die höchste Flottungsanzahl und aufgrund der Rückstelleigenschaft des elastischen Schussfadens ein hoher Kontraktionsanteil gegeben ist, in diesem Bereich zu einem leichten Zusammenziehen, so dass auch an dieser Seite der leichte Knick, siehe Fig. 1, ausgebildet wird.

Abschließend noch der Hinweis, dass die Gestrickarten in den einzelnen Gestrickabschnitten nicht zwingend dem Muster, wie in Fig. 2 gezeigt, entsprechen müssen. So ist es denkbar, die ersten Gestrickabschnitte 5, die grundsätzlich mit einer x : y-Flottung gestrickt sind, wobei x < y ist, anstelle der gezeigten 1 : 3-Flottung auch als 1 : 4-, 2 : 4- oder 2 : 5-Flottung zu stricken. Der zweite Gestrickabschnitt 4, der im Beispiel als 1 : 1-Flottung gestrickt ist, und der grundsätzlich mit einer a : b-Flottung gestrickt wird, wobei a ≤ b ist und b < y ist, kann beispielsweise auch als 2 : 2-Flottung oder als 1 : 2-Flottung gestrickt sein. Wichtig jedoch ist stets, dass die Flottungsanzahl höher ist als im ersten Gestrickabschnitt 5. Bevorzugt ist das Maschenbild derart, dass sich die in Fig. 2 gezeigten einheitlichen Maschenstäbchen 12 ausbilden, so dass es zur beschriebenen Reihen- oder Rippenstruktur kommt.

Die dritten Gestrickabschnitte 5 können auch als 1 : 3- oder als 2 : 4-Flottung gestrickt sein, wichtig hierbei ist jedoch stets der symmetrische Versatz der Flottung von Maschenreihe zu Maschenreihe um eine Nadel, so dass sich die Zick-Zack-Struktur ergibt.

Lediglich der vierte Gestrickabschnitt 7 sollte bevorzugt stets glatt gestrickt sein.

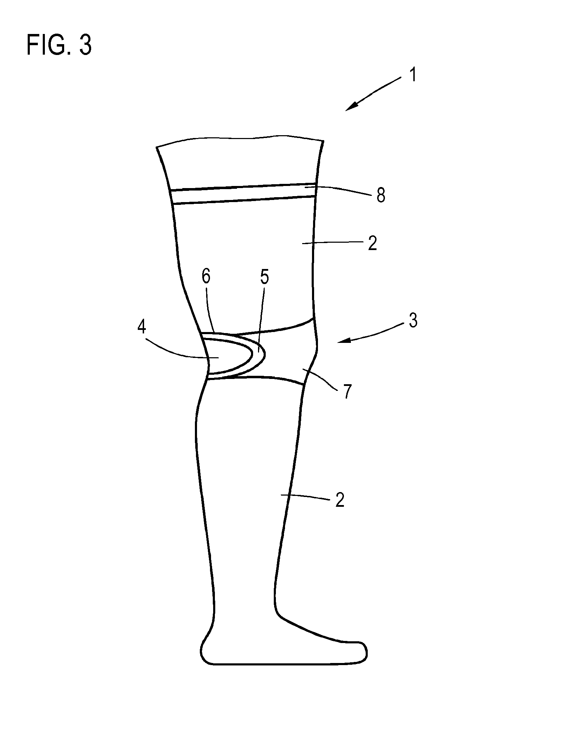

Fig. 3 zeigt schließlich ein weiteres Ausführungsbeispiel eines erfindungsgemäßen Rundgestricks 1, das hier als Beinstrumpf gezeigt ist. Er besteht letztlich aus den gleichen Gestrickabschnitten wie das Rundgestrick 1 aus Fig. 1. Vorgesehen ist also wiederum ein Grundgestrick bestehend aus den ersten Gestrickabschnitten 2, wobei der in Fig. 3 gezeigte untere erste Gestrickabschnitt 2 auch einen Fußteil aufweist, der natürlich, sofern erforderlich, eine spezifische Strickung aufweist.

Zwischen den beiden Gestrickabschnitten 2 ist wiederum ein Gestrickbereich 3 realisiert, der dem aus Fig. 1 entspricht. Er besteht also auch hier aus einem zweiten Gestrickabschnitt 4, der die imitierten Plüsch-, also die leicht erhabene, weiche und anschmiegsame Oberfläche, die zum Bein respektive zur Haut gerichtet ist, aufweist. Er ist auch hier vollständig eingeschlossen von den dritten Gestrickabschnitten 5, die auch hier über die Verbindungsabschnitte 6 miteinander verbunden sind. An diese schließt sich, den Gestrickbereich 3 komplettierend, der vierte Gestrickabschnitt 7 an. Die einzelnen Strickmuster der Gestrickabschnitte entsprechen denen wie bezüglich Fig. 1 beschrieben. Da es sich hier um einen Beinstrumpf handelt, an dem ein Fußbereich angestrickt ist, ist hier nur ein Bündchen 8 vorgesehen.

Ersichtlich ist auch hier der Gestrickbereich 3 so ausgeführt, dass in der Tragstellung der weiche, polsternde zweite Gestrickabschnitt 4 mit seiner plüschartigen, weichen und erhabenen Fläche zur Kniekehle hin gerichtet ist respektive im Kniekehlenbereich angeordnet ist. Der gegenüberliegende, aufgrund der hohen Maschenanzahl gedehnte und vom Gestrickabschnitt 7 gebildete Bereich übergreift den Bereich des Knies. Dieses Rundgestrick kann auch als Beinstrumpf ohne Fußbereich, jedoch mit einem zweiten Bündchen, oder als komplette Strumpfhose ausgeführt sein.

## Patentansprüche

1. Kompressives Rundgestrick (1) zum Überziehen über eine ein Gelenk aufweisende Extremität, aufweisend einen Strickfaden und einen elastischen Faden, die die Maschen bilden, und einen eingelegten elastischen Schussfaden, aufweisend:
- zwei in Gestricklängsrichtung voneinander beabstandete erste Gestrickabschnitte (2), die mit Flottung gestrickt sind, und zwischen denen ein Gestrickbereich (3) bestehend aus weiteren in Umfangsrichtung aneinander anschließenden unterschiedlichen Gestrickabschnitten vorgesehen ist,
- wobei der Gestrickbereich (3) aufweist:
-- einen zweiten Gestrickabschnitt (4), der mit Flottung gestrickt ist, wobei die Flottungsanzahl größer als die eines ersten Gestrickabschnitts (2) ist,
-- zwei in Umfangsrichtung an den zweiten Gestrickabschnitt (4) anschließende dritte Gestrickabschnitte (5), die jeweils mit Flottung gestrickt sind, wobei die Flottungen einer Maschenreihe zur nächsten Maschenreihe versetzt gestrickt sind,
-- sowie einen in Umfangsrichtung zwischen den beiden dritten Gestrickabschnitten (5) vorgesehenen vierten Gestrickabschnitt (7), der glatt gestrickt ist.

2. Kompressives Rundgestrick nach Anspruch 1, **dadurch gekennzeichnet, dass** der zweite Gestrickabschnitt (4) in ovaler Form gestrickt ist.

3. Kompressives Rundgestrick nach Anspruch 1 oder 2, **dadurch gekennzeichnet, dass** die dritten Gestrickabschnitte (5) den zweiten Gestrickabschnitt (4) vollständig umgeben.

4. Kompressives Rundgestrick nach einem der vorangehenden Ansprüche, **dadurch gekennzeichnet, dass** die ersten Gestrickabschnitte (2) mit einer x : y-Flottung gestrickt ist, wobei x < y ist.

5. Kompressives Rundgestrick nach Anspruch 4, **dadurch gekennzeichnet, dass** die ersten Gestrickabschnitte (2) mit einer 1 : 3-Flottung gestrickt sind.

6. Kompressives Rundgestrick nach Anspruch 4 oder 5, **dadurch gekennzeichnet, dass** der zweite Gestrickabschnitt (4) mit einer a : b-Flottung gestrickt ist, wobei a ≤ b ist und b < y ist.

7. Kompressives Rundgestrick nach Anspruch 6, **dadurch gekennzeichnet, dass** der zweite Gestrickabschnitt (4) mit einer 1 : 1-Flottung gestrickt ist.

8. Kompressives Rundgestrick nach einem der vorangehenden Ansprüche, **dadurch gekennzeichnet, dass** die dritten Gestrickabschnitte (5) derart hinsichtlich des Flottungsversatzes gestrickt sind, das sich ein Zick-Zack-Muster ergibt.

9. Kompressives Rundgestrick nach einem der vorangehenden Ansprüche, **dadurch gekennzeichnet, dass** die dritten Gestrickabschnitte (5) mit einer 1 : 2-Flottung gestrickt sind.

10. Kompresives Rundgestrick nach einem der vorangehenden Ansprüche, **dadurch gekennzeichnet, dass** ein Strickfaden aus einer Kunstfaser, insbesondere PA, PES, PP, oder einer Naturfaser, insbesondere Baumwolle oder Seide verwendet ist.

11. Kompressives Rundgestrick nach einem der vorangehenden Ansprüche, **dadurch gekennzeichnet, dass** für die Bildung der ersten bis vierten Gestrickabschnitte (2, 4, 5, 7) Strickfäden mit unterschiedlicher Farbe verwendet sind.

12. Kompressives Rundgestrick nach einem der vorangehenden Ansprüche, **dadurch gekennzeichnet, dass** ein elastischer Faden mit einer Elastanseele, die mit einem Faden aus einer Kunstfaser, insbesondere PA, PES, PP, oder einer Naturfaser, insbesondere Baumwolle oder Seide umwunden ist, verwendet ist.

13. Kompressives Rundgestrick nach einem der vorangehenden Ansprüche, **dadurch gekennzeichnet, dass** ein Schussfaden aus einem Elastomer, insbesondere auf PU-Basis, verwendet ist.

14. Kompressives Rundgestrick nach einem der vorangehenden Ansprüchen, **dadurch gekennzeichnet, dass** an den Endbereichen der ersten Gestrickabschnitte (2) elastische Bündchen (8) angestrickt oder angenäht sind.

15. Kompressives Rundgestrick nach einem der vorangehenden Ansprüche, **dadurch gekennzeichnet, dass** es als Armstrumpf zum Überziehen über den Bereich des Ellebogengelenks oder als Beinstrumpf oder Strumpfhose zum Überziehen über den Bereich des Kniegelenks ausgeführt ist.

## Claims

1. Compressive circular knit(1) for pulling over an articulated extremity, having a knitting thread and an elastic thread, which form the loops, and an inlaid elastic weft thread, comprising:
- two first knitted fabric portions (2) which are spaced apart from each other in the knitted fabric longitudinal direction and which are knitted with floating and wherebetween there is provided a knitted fabric region (3) consisting of further different knitted fabric portions adjoining each other in the circumferential direction,
- wherein the knitted fabric region (3) has:
-- a second knitted fabric portion (4) knitted with floating, wherein the float count is greater than that of a first knitted fabric portion (2),
-- two third knitted fabric portions (5) adjoining the second knitted fabric portion (4) in the circumferential direction which are each knitted with floating, wherein the floats of any one course are knitted offset relative to the next course,
-- and also a fourth knitted fabric portion (7) provided in the circumferential direction between the two third knitted fabric portions (5) and knitted plain.

2. Compressive circular knit according to Claim 1, **characterized in that** the second knitted fabric portion (4) is knitted in an oval shape.

3. Compressive circular knit according to Claim 1 or 2, **characterized in that** the third knitted fabric portions (5) completely surround the second knitted fabric portion (4).

4. Compressive circular knit according to any preceding claim, **characterized in that** the first knitted fabric portions (2) are knitted with an x:y float, where x is < y.

5. Compressive circular knit according to Claim 4, **characterized in that** the first knitted fabric portions (2) are knitted with a 1:3 float.

6. Compressive circular knit according to Claim 4 or 5, **characterized in that** the second knitted fabric portion (4) is knitted with an a:b float, where a is ≤ b and b is < y.

7. Compressive circular knit according to Claim 6, **characterized in that** the second knitted fabric portion (4) is knitted with a 1:1 float.

8. Compressive circular knit according to any preceding claim, **characterized in that** the third knitted fabric portions (5) are knitted with respect to the float offset such that a zigzag pattern is obtained.

9. Compressive circular knit according to any preceding claim, **characterized in that** the third knitted fabric portions (5) are knitted with a 1:2 float.

10. Compressive circular knit according to any preceding claim, **characterized in that** a knitting thread formed from a manufactured fiber, in particular PA, PES, PP, or from a natural fiber, in particular cotton or silk, is used.

11. Compressive circular knit according to any preceding claim, **characterized in that** knitting threads of differing color are used to form the first to fourth knitted fabric portions (2, 4, 5, 7).

12. Compressive circular knit according to any preceding claim, **characterized in that** an elastic thread having an elastane core wrapped with a thread formed from a manufactured fiber, in particular PA, PES, PP, or from a natural fiber, in particular cotton or silk, is used.

13. Compressive circular knit according to any preceding claim, **characterized in that** a weft thread formed from an elastomer, in particular a PU-based elastomer, is used.

14. Compressive circular knit according to any preceding claim, **characterized in that** elastic cuffs (8) are knitted or sewn to the end regions of the first knitted fabric portions (2).

15. Compressive circular knit according to any preceding claim, **characterized in that** it is constructed as arm stocking for pulling over the region of the elbow joint or as leg stocking or pantyhose for pulling over the region of the knee joint.

## Revendications

1. Tricot circulaire compressif (1) destiné à être enfilé sur une extrémité présentant une articulation, présentant un fil à tricoter et un fil élastique, qui forment les mailles, et un fil de trame élastique inséré, présentant:
- deux premières parties de tricot (2) espacées l'une de l'autre dans la direction longitudinale du tricot, qui sont tricotées avec flottage, et entre lesquelles il est prévu une zone de tricot (3) se composant d'autres parties de tricot différentes se raccordant l'une à l'autre en direction périphérique,
- dans lequel la zone de tricot (3) présente
- une deuxième partie de tricot (4), qui est tricotée avec flottage le nombre de flottages étant plus grand que celui d'une première partie de tricot (2),
- deux troisièmes parties de tricot (5) se raccordant en direction périphérique à la deuxième partie de tricot (4), qui sont respectivement tricotées avec flottage, les flottages d'une rangée de mailles étant tricotés en décalage par rapport à la rangée de mailles voisine,
- ainsi qu'une quatrième partie de tricot (7), qui est tricotée à plat, prévue en direction périphérique entre les deux troisièmes parties de tricot (5).

2. Tricot circulaire compressif selon la revendication 1, **caractérisé en ce que** la deuxième partie de tricot (4) est tricotée sous forme ovale.

3. Tricot circulaire compressif selon la revendication 1 ou 2, **caractérisé en ce que** les troisièmes parties de tricot (5) entourent entièrement la deuxième partie de tricot (4).

4. Tricot circulaire compressif selon l'une quelconque des revendications précédentes, **caractérisé en ce que** les premières parties de tricot (2) sont tricotées avec un flottage x:y, pour lequel x < y.

5. Tricot circulaire compressif selon la revendication 4, **caractérisé en ce que** les premières parties de tricot (2) sont tricotées avec un flottage 1:3.

6. Tricot circulaire compressif selon la revendication 4 ou 5, **caractérisé en ce que** la deuxième partie de tricot (4) est tricotée avec un flottage a:b, pour lequel a ≤ b et b < y.

7. Tricot circulaire compressif selon la revendication 6, **caractérisé en ce que** la deuxième partie de tricot (4) est tricotée avec un flottage 1:1.

8. Tricot circulaire compressif selon l'une quelconque des revendications précédentes, **caractérisé en ce que** les troisièmes parties de tricot (5) sont tricotées, en ce qui concerne le décalage du flottage, de telle manière qu'il en résulte un motif en zigzag.

9. Tricot circulaire compressif selon l'une quelconque des revendications précédentes, **caractérisé en ce que** les troisièmes parties de tricot (5) sont tricotées avec un flottage 1:2.

10. Tricot circulaire compressif selon l'une quelconque des revendications précédentes, **caractérisé en ce que** l'on a utilisé un fil à tricoter en une fibre synthétique, en particulier en PA, PES, PP, ou en une fibre naturelle, en particulier en coton ou en soie.

11. Tricot circulaire compressif selon l'une quelconque des revendications précédentes, **caractérisé en ce que** l'on a utilisé pour la formation des premières aux quatrièmes parties de tricot (2, 4, 5, 7) des fils à tricoter de différentes couleurs.

12. Tricot circulaire compressif selon l'une quelconque des revendications précédentes, **caractérisé en ce que** l'on a utilisé un fil élastique avec une âme en élasthanne, autour de laquelle est enroulé un fil en une fibre synthétique, en particulier en PA, PES, PP, ou en une fibre naturelle, en particulier en coton ou en soie.

13. Tricot circulaire compressif selon l'une quelconque des revendications précédentes, **caractérisé en ce que** l'on a utilisé un fil de trame en un élastomère, en particulier à base de PU.

14. Tricot circulaire compressif selon l'une quelconque des revendications précédentes, **caractérisé en ce que** des bandelettes élastiques (8) sont tricotées ou cousues sur les régions d'extrémité des premières parties de tricot (2).

15. Tricot circulaire compressif selon l'une quelconque des revendications précédentes, **caractérisé en ce qu'**il est réalisé sous forme de manche à enfiler sur la région de l'articulation du coude ou sous forme de bas ou de collant à enfiler sur la région de l'articulation du genou.
